# EUROPEAN PATENT APPLICATION

(11) **EP 1 837 403 A2**
(43) Date of publication of application: **26.09.2007**
(21) Application number: 05850035.6
(22) Date of filing: 23.12.2005
(51) Int. Cl.: C12Q 1/68

(54) **PROBES AND METHODS FOR THE SIMULTANEOUS DETECTION AND IDENTIFICATION OF MULTIPLE VIRUSES THAT CAUSE RESPIRATORY INFECTIONS IN HUMANS**

(30) Priority: 24.12.2004 ES 200403083
(71) Applicant: Instituto de salud carlos III, E-28029 Madrid (ES)
(72) Inventor: PEREZ BREÑA, Pilar, 28029 Madrid (ES); COIRAS LOPEZ, Maria Teresa, 28029 Madrid (ES); CASAS FLECHA, Inmaculada, 28029 Madrid (ES)
(74) Representative: Maldonado Jordan, Julia
(86) International application number: PCT/ES2005/000704
(87) International publication number: WO 2006/070034

(57) **Abstract**

The invention relates to probes and assays which are used for the simultaneous detection, in a single assay sample, of a plurality of nucleic acid sequences of viruses that cause respiratory infections in humans, selected from among influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof.

LIST OF SEQUENCES

<110> INSTITUTO DE SALUD CARLOS III

<120> PROBES AND METHODS FOR THE SIMULTANEOUS DETECTION AND
IDENTIFICATION OF MULTIPLE VIRUSES THAT CAUSE RESPIRATORY
INFECTIONS IN HUMANS

<160> 19

<210> 1
<211> 23
<212> DNA
<213> Artificial sequence

<223> probe for detecting the presence of influenza virus type
A by means of hybridization

<400> 1
cccagratgt gctctctgat gca                                23

<210> 2
<211> 22
<212> DNA
<213> Artificial sequence

<223> probe for detecting the presence of influenza virus type
A by means of hybridization

<400> 2
catgccatcc ayaccarytg rc                                 22

<210> 3
<211> 23

<212> DNA
<213> Artificial sequence

<223> probe for detecting the presence of influenza virus type
B by means of hybridization

<400> 3
cagatgatgg tcaaagctgg act                                              23

<210> 4
<211> 23
<212> DNA
<213> Artificial sequence

<223> probe for detecting the presence of influenza virus type
B by means of hybridization

<400> 4
tcttcwatgt ctgcaatccc tgg                                              23

<210> 5
<211> 23
<212> DNA
<213> Artificial sequence

<223> probe for detecting the presence of influenza virus type
C by means of hybridization

<400> 5
cgatgatgaa attgaatcaa tag                                              23

<210> 6
<211> 23
<212> DNA
<213> Artificial sequence

<223> probe for detecting the presence of human respiratory
syncytial virus type A by means of hybridization

<400> 6
gtcaaaacat cactgaagaa ttt                                    23

<210> 7
<211> 23
<212> DNA
<213> Artificial sequence

<223> probe for detecting the presence of human respiratory
syncytial virus type A by means of hybridization

<400> 7
ttacattggt wtttttggya ttg                                    23

<210> 8
<211> 23
<212> DNA
<213> Artificial sequence

<223> probe for detecting the presence of human respiratory
syncytial virus type B by means of hybridization

<400> 8
tgaggtgagg tacaatgcat taa                                    23

<210> 9
<211> 23
<212> DNA
<213> Artificial sequence

<223> probe for detecting the presence of human adenovirus by

means of hybridization

<400> 9
ccmgcrttrc ggtgrtggtt raa                                              23

<210> 10
<211> 23
<212> DNA
<213> Artificial sequence

<223> probe for detecting the presence of human parainfluenza
virus type 1 by means of hybridization

<400> 10
taaattcaga tatgtatcct gat                                              23

<210> 11
<211> 23
<212> DNA
<213> Artificial sequence

<223> probe for detecting the presence of human parainfluenza
virus type 2 by means of hybridization

<400> 11
tacctaagtg atggaatcaa tcg                                              23

<210> 12
<211> 23
<212> DNA
<213> Artificial sequence

<223> probe for detecting the presence of human parainfluenza
virus type 3 by means of hybridization

<400> 12
acagatgtat atcaactgtg ttc                                              23

<210> 13
<211> 23
<212> DNA
<213> Artificial sequence

<223> probe for detecting the presence of human parainfluenza
virus types 4A and 4B by means of hybridization

<400> 13
atggtgaaaa gaacatggag att                                              23

<210> 14
<211> 20
<212> DNA
<213> Artificial sequence

<223> probe for detecting the presence of enterovirus by means
of hybridization

<400> 14
tggctgctta tggtgacaat                                                  20

<210> 15
<211> 20
<212> DNA
<213> Artificial sequence

<223> probe for detecting the presence of rhinovirus by means
of hybridization

<400> 15
   gggaygggac cractacttt                                                   20

<210> 16
<211> 20
<212> DNA
<213> Artificial sequence

<223> probe for detecting the presence of human coronavirus
type 229E by means of hybridization

<400> 16
ggacttggca ctgtggacgc                                                20

<210> 17
<211> 20
<212> DNA
<213> Artificial sequence

<223> probe for detecting the presence of human coronavirus
type OC43 by means of hybridization

<400> 17
atcagatcag tggatacact                                                20

<210> 18
<211> 27
<212> DNA
<213> Artificial sequence

<223> probe for detecting the presence of coronavirus that
causes severe acute respiratory syndrome (SARS) by means of
hybridization

<400> 18
tggattggct ttgatgtaga gggctgt                                        27

<210> 19
<211> 19
<212> DNA
<213> Artificial sequence

<223> probe for detecting the presence of human
metapneumovirus by means of hybridization

<400> 19
ctgttaatat cccacacca 19

## Description

### FIELD OF THE INVENTION

The invention generally relates to the detection of nucleic acid sequences, and particularly to probes and assays useful for simultaneously detecting a plurality of nucleic acid sequences in a single assay sample. More specifically, the invention relates to probes and methods for the detection, by means of hybridization, of a plurality of nucleic acid sequences of viruses that cause respiratory infections in humans, eventually present in a single assay sample, selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof.

### BACKGROUND OF THE INVENTION

The signs and symptoms causing respiratory tract infection due to different viruses and even some bacteria are generally indistinguishable from a clinical point of view. It is therefore necessary to provide a diagnostic tool which allows not only identifying the agent causing the infection but also that this identification be carried out in a short time period so that the obtained result contributes to quickly implementing the suitable curative or palliative measures and, in the event that the infection is caused by a virus, so that the unnecessary administration of antibiotics, with the possible occurrence of resistant bacteria, is prevented. Furthermore, having a quick and reliable method of diagnosing the viral infection is extremely important for those viral diseases which can be treated by means of the administration of antiviral drugs, as is the case of flu viruses. This would significantly contribute to reducing the morbi-mortality caused by these viruses in groups at risk, such as the elderly, infants and immunodepressed patients. Considering that new antiviral drugs may be available in the near future, there will also be a growing demand for sensitive diagnostic methods which are routinely included in microbiology laboratories that allow quickly identifying the different viruses that cause respiratory infections.

The laboratory diagnosis is therefore essential for the detection and identification of viruses that cause respiratory tract infections which cause diseases with clinically indistinguishable signs and symptoms. It is mistakenly believed, for example, that streptococcal pharyngitis can be reliably differentiated from viral pharyngitis in an individual patient. A probabilistic estimate can even be carried out (for example, the symptoms of a common cold have an 80% negative predictive value for the diagnosis of bacterial pharyngitis), but the confirmation must be carried out by means of laboratory analysis.

Generally the etiological diagnosis of viral respiratory infections in humans has been carried out, up until now, by using traditional methods, such as isolation in cell cultures or the indirect immunofluorescence (IF) technique which, though they have a high sensitivity, they only allow, except on very few occasions, distinguishing the presence of more than one virus coinfecting the sample, which may be due to several factors, for example: (i) the destruction of the cell monolayer by the virus having higher cytopathic activity, which would mask the growth of other viruses possibly present in the sample to be assayed which grew more slowly or which caused a less evident cytopathic effect, and (ii) the non-availability of specific monoclonal antibodies which allowed the identification of the viruses coinfecting the sample. Therefore, the diagnosis of coinfections is undervalued due to the absence of techniques that are sensitive and specific enough to detect them.

Routine assays based on the detection of the viral genome in a clinical sample or in a cell culture thereof are increasingly being introduced today in routine clinical diagnosis. The types of assays which allow detecting viral genomes are generally based on hybridization methods or on amplification methods.

In the first case (hybridization methods), viral genomes can be detected by means of the use of probes or oligonucleotides with a homologous sequence to that of the virus possibly present in the sample to be analyzed. These techniques are usually simple and use non-radioactive developing, for example by means of fluorescence or chemiluminescence. Methods based on hybridization techniques further have the advantages of specificity and sensitivity, the possibility of being able to quantify the amount of viral nucleic acids present in the sample (viral load), which can be used to monitor the response of the patient to antiviral therapy.

Genome amplification methods allow amplifying several million fold certain viral genome sequences such that they can be easily detected. Polymerase strand reaction (PCR) in both the simple and multiple formats has proven to be an extremely sensitive and specific genome amplification method in the diagnosis of respiratory infections caused by viruses. Genome amplification is relatively common in assays for diagnosing respiratory infections in humans due to the fact that, in general, the amount of virus present in a conventional respiratory sample (nasopharyngeal lavage or suction, pharyngeal smear, gargling, etc.) is usually scarce. Therefore, to obtain maximum sensitivity it is usually necessary to carry out, after the first amplification reaction, a second amplification on the fragment obtained by means of, for example, nested-PCR. Generally, the first amplification reaction is RT-PCR (RT: retrotranscription or reverse transcription) since most of the viruses that cause respiratory infections in humans have a genome formed by RNA. Even though carrying out nested-PCR is simple and easy to introduce in routine procedures in a diagnostic laboratory, it also involves a possible source of false positives due to the fact that the enormous amplification of the viral gene fragment may give rise to contaminations due to amplification products of adjacent samples. So in order to routinely carry out nested-PCR techniques it is necessary to take special precautions, including, for example, the fact that the steps necessary for carrying out the amplification process must be carried out in physically separate laboratories, in different laminar flow cabins, each of them provided with reagents, sets of micropipettes, sterile tubes and tips with sterile filters, all completely independent. In the case of small laboratories or laboratories provided with scarce laboratory material and/or equipment, this is usually not very plausible.

In addition, the methods based on amplification reactions allow the detection of one or several viral agents simultaneously, but not of all the possible viruses that cause respiratory infections because, as is well known, there are technical difficulties for carrying out multiple PCRs allowing the simultaneous detection of several viruses, such as the loss of sensitivity by increasing the number of primers in the reaction mixture or the need to adjust the location of the primers in the gene to be amplified for the purpose of obtaining easily distinguishable viruses due to their size upon analyzing them by means of agarose gel electrophoresis.

Nevertheless, a multiple RT-PCR has been described for the simultaneous detection and typing of some viruses that cause respiratory infections in humans, specifically A, B and C influenza viruses, A and B respiratory syncytial virus and adenovirus (Coiras et al., 2003, J Med Virol 69:132-144).

An interesting alternative approach to the diagnosis of respiratory infections of a viral origin is based on the combined application of genome amplification and hybridization techniques with specific probes. This approach is the one followed in the "Hexaplex" assay, which is based on amplification by means of multiple RT-PCR in a hybridization system with probes and enzymatic detection. It is possible to detect the following viruses by means of this technique: types A and B respiratory syncytial virus, types A and B influenza virus and types 1, 2 and 3 parainfluenza virus. Nevertheless, this method does not allow detecting other important viruses that cause respiratory infections in humans such as: adenovirus, an important group of pathogens of the respiratory tract; type 4 parainfluenza virus, generally causing infections in children under the age of two which are not as mild as had been described previously; enterovirus, rhinovirus, or coronavirus, these last two groups being the most common and possible pathogens of the upper respiratory tract causing serious complications in the patient. Furthermore, these last three groups of viruses are found repeatedly in clinical samples of patients with respiratory infection, but their role as agents causing respiratory infections is rather undefined, as well as their epidemiology due to the difficulty of detecting them by means of standard methods.

Therefore, the current methods used in the routine laboratory diagnosis of viruses that cause respiratory infections in humans have several disadvantages:
- scarce sensitivity and/or specificity;
- occurrence of false negatives when techniques based on IF are used;
- occurrence of false positives when techniques based on nested-PCR are used;
- days or even weeks can go by before obtaining results, especially in the case of viral isolation in cell cultures;
- dependence of some techniques on the condition of the clinical sample, result of the sampling, storage and transport process thereof. This is especially important in the case of viral isolation in cell cultures and of immunofluorescence techniques in which false negatives may occur;
- contamination of the sample with bacteria from the normal flora of the area where the sampling thereof has been carried out may make the analysis by means of methods such as viral isolation in cell cultures difficult, which can cause false negatives; and
- the need, in some cases, of qualified or experienced personnel in the issue at hand.

Therefore there is a need to develop effective methods which allow simultaneously detecting and identifying the main viral agents causing respiratory infections in humans which solve all or part of the problems existing with the currently used methods in the routine laboratory diagnosis of viruses that cause respiratory infections in humans. Said methods must provide, in a quick, specific and sensitive manner, results which can be used by the clinic in making therapeutic, prophylactic or palliative decisions. By way of illustration, a suitable diagnostic method would be one that allows differentiating viruses that cause respiratory infections which require immediate hospitalization or isolation of the patient, from other milder causes of respiratory tract infection. Advantageously, said methods must be carried out in laboratories close to where the patient is located, with inexpensive facilities and equipment and in which the participation of highly qualified personnel is not necessary.

### SUMMARY OF THE INVENTION

The invention provides a solution to the existing need consisting of probes and methods which allow simultaneously detecting and identifying, in a quick, specific and sensitive manner, the main viruses that cause respiratory infections in humans possibly present in a single assay sample. To that end the invention is based on the development of a hybridization system in which different oligonucleotides with specific sequences (probes) are immobilized on a solid support. These probes have been designed to specifically hybridize with target sequences present in the genome of said viruses, which allows not only the detection and identification of the virus that causes the respiratory disease, but which further simultaneously enables its typing. This system can be adapted to the development of easy-to-handle kits intended for microbiology laboratories which require establishing an etiological diagnosis of infectious respiratory diseases in humans.

Viruses that cause respiratory infections in humans that can be identified by means of the method provided by this invention include influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof.

Therefore, in one aspect, the invention relates to a probe useful for identifying viruses that cause respiratory infections in humans selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS) and human metapneumovirus.

In another aspect, the invention relates to a hybridization platform comprising a solid support and at least one of said probes immobilized on said solid support.

In another aspect, the invention relates to a method for identifying viruses that cause respiratory infections in humans selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof, based on the hybridization of target sequences in the genome of said viruses, optionally amplified by means of an amplification reaction, with said probes.

In another aspect the invention relates to a kit for identifying viruses that cause respiratory infections in humans selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof, by means of said method comprising at least one of said probes, or at least one of said hybridization platforms.

### BRIEF DESCRIPTION OF THE FIGURES

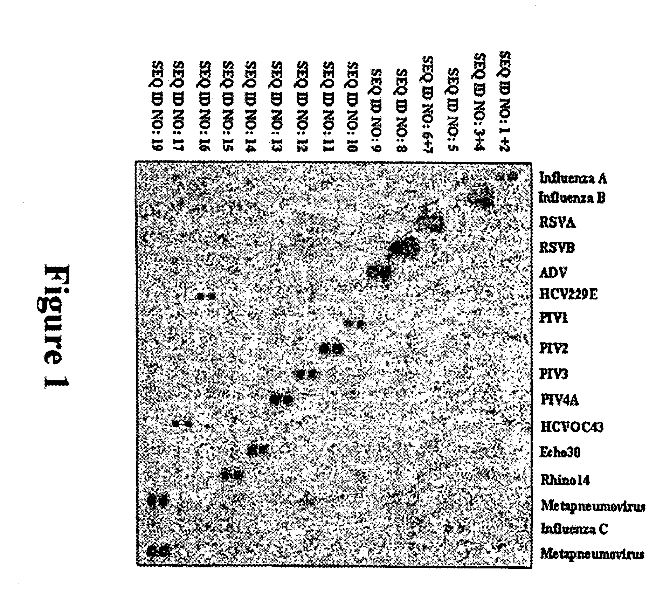

Figure 1 shows the detection of reference strains of viruses that cause respiratory infections in humans grown in cell cultures by means of hybridization in nylon membrane and viewing by means of chemiluminescence. The assayed viruses were influenza virus type A (IVA), influenza virus type B (IVB), human respiratory syncytial virus type A (RSVA), human respiratory syncytial virus type B (RSVB), human adenovirus (ADV1), human coronavirus type 229E (HCV229E), human parainfluenza virus type 1 (PIV1), human parainfluenza virus type 2 (PIV2), human parainfluenza virus type 3 (PIV3), human parainfluenza virus type 4A (PIV4A), human coronavirus type OC43 (HCVOC43), echovirus (Echo30), rhinovirus (Rhinol4), human metapneumovirus (metapneumovirus) and influenza virus type C (IVC). For more information see Example 1.

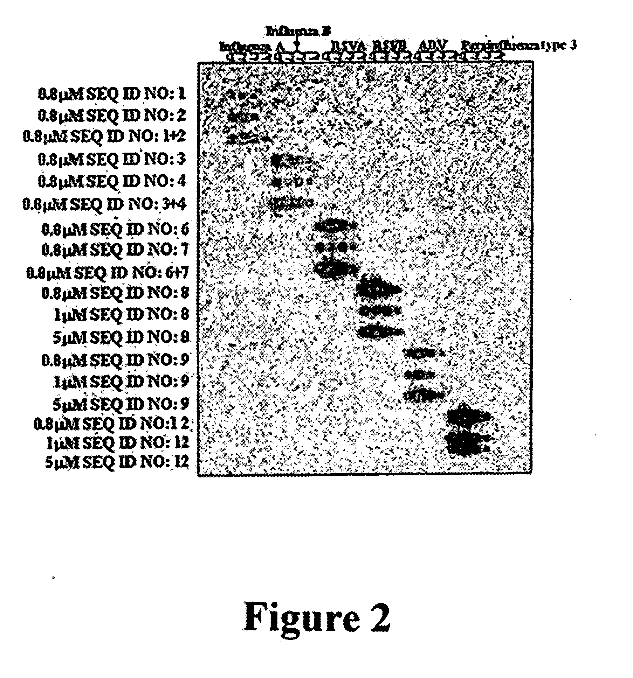

Figure 2 shows the detection by means of hybridization on nylon membrane and viewing by means of chemiluminescence of viruses that cause respiratory infections in humans, isolated from clinical samples of the respiratory tract inoculated in cell cultures. The detected viruses were influenza virus type A (Influenza A), influenza virus type B (Influenza B), respiratory syncytial virus type A (RSVA), respiratory syncytial virus type B (RSVB), human adenovirus (ADV1) and type 3 parainfluenza virus (Parainfluenza type 3). Probes with different concentrations were used for the purpose of observing the sensitivity according to the concentration of probe. For more information see Example 2.

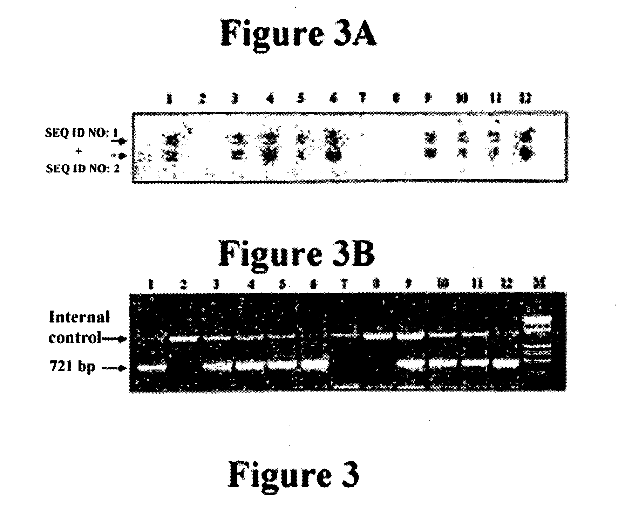

Figure 3 shows the detection of influenza virus type A present in clinical samples by means of hybridization on nylon membrane (Figure 3A) and by means of nested-PCR (Figure 3B). See Example 3 for a description of the assay. Figure 3A shows the results of the hybridization of nucleic acids from the assayed clinical samples for detecting influenza virus type A with the probes (SEQ ID NO: 1 and SEQ ID NO: 2) immobilized on the membrane (Lanes 1-12). Influenza virus A was not detected in samples 2, 7 and 8. Figure 3B shows the results of a nested-PCR carried out to check the sensitivity and specificity of the prior technique (hybridization). Lanes 1-12 correspond to lanes 1-12 seen in Figure 3A. Lane M corresponds to molecular weight marker XIV supplied by Roche Diagnostics (100 bp marker). A band of 721 base pairs (bp) corresponding to the amplified fragment of the gene of the nucleoprotein of type A human influenza virus and a band of 837 bp corresponding to the internal control used as a control of the process of extracting the genetic material from the sample and of the PCR reaction (see Example 3) can be seen in all the lanes (except 2, 7 and 8). It can be seen that the results obtained with both techniques are consistent.

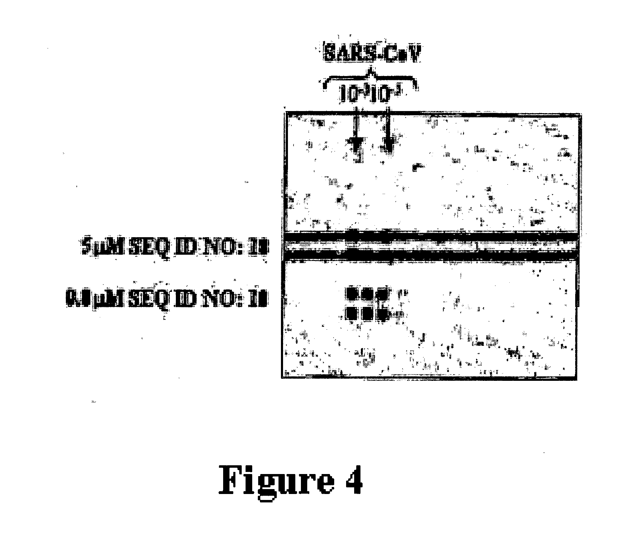

Figure 4 shows the detection of coronavirus that causes severe acute respiratory syndrome (SARS) present in clinical samples by means of hybridization on nylon membrane and viewing by chemiluminescence. Several concentrations of the probe used (SEQ ID NO: 18) were assayed. See Example 4 for a description of the assay.

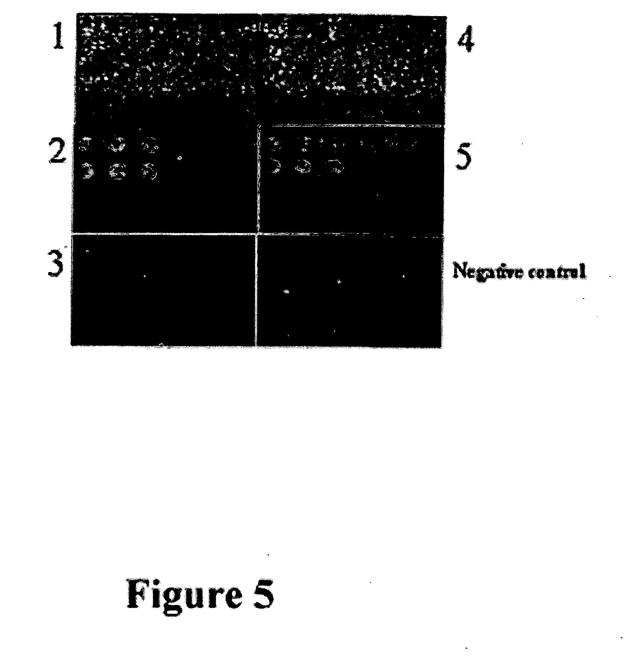

Figure 5 shows the detection of human adenovirus in clinical samples (respiratory secretions) from patients with respiratory symptoms by means of hybridization on a glass support (slide) and viewing by fluorescence. The fluorescence emitted by the Cy3 molecules present in the amplification product obtained by means of initiating oligonucleotides specific for human adenovirus can be seen. Dilutions of adenovirus that causes respiratory infection in humans belonging to serotypes 1 (image 1), 2 (image 2), 4 (image 3), 5 (image 4) and 7 (image 5) have been used for the amplification and labeling by means of PCR, these dilutions being equivalent to 10³ copies of a plasmid containing the fragment of the gene of the human adenovirus hexon amplified under the conditions described in Example 5. A negative control is included.

### DETAILED DESCRIPTION OF THE INVENTION

The invention provides probes and methods for simultaneously identifying a plurality of nucleic acid sequences of the main viruses that cause respiratory infections in humans possibly present in a single assay sample.

### Probes

In one aspect, the invention relates to probes, hereinafter probes of the invention, which allow the identification, by means of specific hybridization, of the main viruses that cause respiratory infections in humans, including influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS) and human metapneumovirus.

The term "probe" as it is used herein refers to an oligonucleotide having a defined sequence able to hybridize with a complementary sequence of a nucleic acid, whereby it can be used to detect and identify complementary, or substantially complementary, sequences in nucleic acids by means of specific hybridization. Likewise, as it is herein used the term "hybridization" refers to the formation of a duplex-type structure by two nucleic acids due to complementary base pairing. Hybridization can occur between completely complementary nucleic acid strands or between "substantially complementary" nucleic acid strands containing small mismatched regions. The conditions under which completely complementary nucleic acid strands hybridize are referred to as "strict hybridization conditions" or "sequence-specific hybridization conditions". Nevertheless, substantially complementary stable double nucleic acid strands can be obtained under less strict hybridization conditions, in which case the tolerated degree of mismatching can be adjusted by means of the suitable adjustment of the hybridization conditions. A person skilled in the art can empirically determine the stability of a duplex taking several variables into account, such as the length and concentration of base pairs of the probes, the ionic force and incidence of the mismatched base pairs, according to the guidelines of the state of the art (see, for example, Sambrook et al., Molecular Cloning: A Laboratory Manual, Cold Spring Harbor Laboratory, Cold Spring Harbor, N.Y. (1989); and Wetmur, Critical Reviews in Biochem. and Mol. Biol. 26(3/4):227-259(1991)).

The probes of the invention are complementary or substantially complementary to specific target sequences contained in the genomes of said viruses that cause respiratory infections in humans (influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS) and human metapneumovirus) such that they allow the generic detection thereof.

The length of the probes of the invention can vary within a broad range, however for practical reasons probes having a small length are preferred, typically comprised between 15 bases and 30 bases, preferably between 19 bases and 23 bases. The use of probes having a larger or smaller length would not affect the sensitivity or specificity of the technique, but it could require carrying out a series of modifications on the conditions under which the technique is carried out by varying the fusion temperature thereof and their GC content, which would basically affect the hybridization temperature and time.

In a particular embodiment, the invention proposes probes for the detection and identification of influenza virus type A by means of specific hybridization including polynucleotides having 15 to 30 bases able to hybridize under strict hybridization conditions with a nucleic acid sequence located in the gene of the nucleoprotein or its complementary strand. Illustrative examples of said probes include the polynucleotides identified as SEQ ID NO: 1 and SEQ ID NO: 2. Said probes can be used separately or together to increase detection sensitivity.

In another particular embodiment, the invention provides probes for the detection and identification of influenza virus type B by means of specific hybridization including polynucleotides having 15 to 30 bases able to hybridize under strict hybridization conditions with a nucleic acid sequence located in the gene of the nucleoprotein or its complementary strand. Illustrative examples of said probes include the polynucleotides identified as SEQ ID NO: 3 and SEQ ID NO: 4. Said probes can be used separately or together to increase detection sensitivity.

In other particular embodiment, the invention provides probes for the detection and identification of influenza virus type C by means of specific hybridization including polynucleotides having 15 to 30 bases able to hybridize under strict hybridization conditions with a nucleic acid sequence located in the gene of the nucleoprotein or its complementary strand. Illustrative examples of said probes include the polynucleotide identified as SEQ ID NO: 5.

In another particular embodiment, the invention provides probes for the detection and identification of human respiratory syncytial virus type A by means of specific hybridization including polynucleotides having 15 to 30 bases able to hybridize under strict hybridization conditions with a nucleic acid sequence located in the gene of the fusion protein (F protein) or its complementary strand. Illustrative examples of said probes include the polynucleotides identified as SEQ ID NO: 6 and SEQ ID NO: 7. Said probes can be used separately or together to increase detection sensitivity.

In another particular embodiment, the invention provides probes for the detection and identification of human respiratory syncytial virus type B by means of specific hybridization including polynucleotides having 15 to 30 bases able to hybridize under strict hybridization conditions with a nucleic acid sequence located in the gene of the fusion protein (F protein) or its complementary strand. Illustrative examples of said probes include the polynucleotide identified as SEQ ID NO: 8.

In another particular embodiment, the invention provides probes for the detection and identification of human adenovirus by means of specific hybridization including polynucleotides having 15 to 30 bases able to hybridize under strict hybridization conditions with a nucleic acid sequence located in the gene of the hexon protein or its complementary strand. Illustrative examples of said probes include the polynucleotide identified as SEQ ID NO: 9.

In another particular embodiment, the invention provides probes for the detection and identification of human parainfluenza virus type 1 by means of specific hybridization including polynucleotides having 15 to 30 bases able to hybridize under strict hybridization conditions with a nucleic acid sequence located in the gene of the hemagglutinin protein or its complementary strand. Illustrative examples of said probes include the polynucleotide identified as SEQ ID NO: 10.

In another particular embodiment, the invention provides probes for the detection and identification of human parainfluenza virus type 2 by means of specific hybridization including polynucleotides having 15 to 30 bases able to hybridize under strict hybridization conditions with a nucleic acid sequence located in the gene of the hemagglutinin protein or its complementary strand. Illustrative examples of said probes include the polynucleotide identified as SEQ ID NO: 11.

In another particular embodiment, the invention provides probes for the detection and identification of human parainfluenza virus type 3 by means of specific hybridization including polynucleotides having 15 to 30 bases able to hybridize under strict hybridization conditions with a nucleic acid sequence located in the gene of the hemagglutinin protein or its complementary strand. Illustrative examples of said probes include the polynucleotide identified as SEQ ID NO: 12.

In another particular embodiment, the invention provides probes for the detection and identification of human parainfluenza virus types 4A and 4B by means of specific hybridization including polynucleotides having 15 to 30 bases able to hybridize under strict hybridization conditions with a nucleic acid sequence located in the gene of the hemagglutinin protein or its complementary strand. Illustrative examples of said probes include the polynucleotide identified as SEQ ID NO: 13, which allows detecting the presence of types 4A or 4B human parainfluenza virus but does not allow distinguishing them.

In another particular embodiment, the invention provides probes for the detection and identification of enterovirus by means of specific hybridization including polynucleotides having 15 to 30 bases able to hybridize under strict hybridization conditions with a nucleic acid sequence located in 5' noncoding region or its complementary strand. Illustrative examples of said probes include the polynucleotide identified as SEQ ID NO: 14.

In another particular embodiment, the invention provides probes for the detection and identification of rhinovirus by means of specific hybridization including polynucleotides having 15 to 30 bases able to hybridize under strict hybridization conditions with a nucleic acid sequence located in 5' noncoding region or its complementary strand. Illustrative examples of said probes include the polynucleotide identified as SEQ ID NO: 15.

In another particular embodiment, the invention provides probes for the detection and identification of human coronavirus type 229E by means of specific hybridization including polynucleotides having 15 to 30 bases able to hybridize under strict hybridization conditions with a nucleic acid sequence located in the gene of the spike protein or its complementary strand. Illustrative examples of said probes include the polynucleotide identified as SEQ ID NO: 16.

In another particular embodiment, the invention provides probes for the detection and identification of human coronavirus type OC43 by means of specific hybridization including polynucleotides having 15 to 30 bases able to hybridize under strict hybridization conditions with a nucleic acid sequence located in the gene of the spike protein or its complementary strand. Illustrative examples of said probes include the polynucleotide identified as SEQ ID NO: 17.

In another particular embodiment, the invention provides probes for the detection and identification of coronavirus that causes the severe acute respiratory syndrome (SARS) by means of specific hybridization including polynucleotides having 15 to 30 bases able to hybridize under strict hybridization conditions with a nucleic acid sequence located in the gene of the spike protein or its complementary strand. Illustrative examples of said probes include the polynucleotide identified as SEQ ID NO: 18.

In another particular embodiment, the invention provides probes for the detection and identification of human metapneumovirus by means of specific hybridization including polynucleotides having 15 to 30 bases able to hybridize under strict hybridization conditions with a nucleic acid sequence located in the gene of the polymerase protein or its complementary strand. Illustrative examples of said probes include the polynucleotide identified as SEQ ID NO: 19.

The probes provided by this invention allow carrying out the simultaneous detection and identification of the main viruses that cause respiratory infections in humans and even their simultaneous typing and/or subtyping.

The probes of the invention, particularly the probes the nucleotide sequences of which are shown in SEQ ID NO: 1- SEQ ID NO: 19, have several advantages since their short length (23 bases at most) make their synthesis very economical and they further allow the generic detection of the main viruses that cause respiratory infections in humans, including serotypes thereof, such as the influenza virus of all subtypes of type A, all the variants of influenza virus type B, virus variants of type C influenza, human respiratory syncytial virus type A, human respiratory syncytial virus type B, all types of human adenovirus, human parainfluenza virus type 1, human parainfluenza virus type 2, human parainfluenza virus type 3, human parainfluenza virus types 4A and 4B, enterovirus, rhinovirus, variants of the human coronavirus type 229E, human coronavirus type OC43, coronavirus that causes severe acute respiratory syndrome (SARS) and variant strains of human metapneumovirus. The use of said probes thus allows detecting and identifying hundreds of viruses that cause respiratory infections with a low cost, therefore forming an important step for producing a diagnostic tool which brings together all the advantages of the hybridization techniques for the simultaneous detection and identification of the main viruses that cause infection in the human respiratory tract.

Other advantages associated with the use of oligonucleotide probes for the detection and identification of viruses that cause respiratory infections in humans, such as the probes of the invention, include:
- the possibility of not only identifying and characterizing the viruses that cause respiratory infections, but also knowing if they are sensitive or resistant to the available antiviral drugs;
- hybridization assays by means of oligonucleotide probes also allow quantifying the viral load in the sample to be analyzed for the purpose of the real time monitoring of the efficacy of the antiviral therapy in the patient; and
- they can be used for real time epidemiological studies due to their quick simultaneous detection and typing of multiple viruses at the same time.

The probes of the invention are advantageously immobilized on a solid support, such as the one described below, to carry out the simultaneous detection and identification of the main viruses that cause respiratory infections in humans and even their simultaneous typing and/or subtyping. Said solid supports in which the probes of the invention have been immobilized, forming hybridization platforms, represent an additional aspect of this invention.

For the purpose of facilitating the immobilization of the probes to the solid support, the probes can incorporate additional features which allow the immobilization to the support but which do not alter their main feature (hybridizing with complementary nucleic acid sequences). By way of illustration, said probes can contain an amino group at end 5' to facilitate the immobilization of the probe and spatially order the probe on the membrane, or in the case of a glass support, the treatment of the surface with poly-L-lysine, allows the immobilization of the probes without spatial ordering.

### Hybridization platform

In another aspect, the invention relates to a hybridization platform comprising a solid support and at least one probe of the invention immobilized on said solid support.

As it is used in this description, the term "solid support" relates to an insoluble material, or to a material which can become insoluble by means of a subsequent reaction. Said solid support can be chosen according to its intrinsic ability to immobilize an oligonucleotide probe, or it can incorporate an additional receptor with the ability to immobilize the oligonucleotide probe such that it allows indirect binding of the probe to the solid support through said receptor. Said additional receptor can be a substance loaded with load opposite to that of the probe or it can be a specific binding member immobilized on (or bound to) the solid support with the ability to immobilize the probe through a specific binding reaction, for example, avidin or streptavidin which allows the immobilization of biotinylated oligonucleotide probes. The solid support can be formed by any suitable material allowing the direct or indirect immobilization of the probe to said solid support, for example, plastics, derivative plastics, polymers, latex, optical fiber, glass or silicon surfaces, ceramic surfaces, metallic surfaces, etc. Said materials can be used in any suitable configuration known by persons skilled in the art, for example, in the form of membranes, sheets, films, plates, chips, etc., or they can completely or partially cover or be bound to inert carriers such as paper, nylon, glass, plastic films, fabrics, etc. Additionally, if desired the solid support can be coated to facilitate the immobilization of the probes to its surface. In a particular embodiment, said solid support is a nylon membrane or a glass surface, for example, a slide, optionally coated, for example, with membranes, silicon supports, polymer supports, etc.

In a particular embodiment, the probe of the invention immobilized on said solid support is selected from the group consisting of the polynucleotides identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ 5 ID NO: 15, SEQ ID NO: 16, SEQ ID NO: 17, SEQ ID NO: 18, SEQ ID NO: 19 and mixtures thereof. Even though the hybridization platform can contain a variable number of probes of the invention immobilized on the solid support, in a particular embodiment, the hybridization platform of the invention comprises 1, 2, 3, 4, 5, 6, 7, 8, 9, 10, 11, 12, 13, 14, 15, 16, 17, 18 or 19 of said probes (SEQ ID NO: 1 - SEQ ID NO: 19). Advantageously, the hybridization platform of the invention comprises said 19 probes of the invention (SEQ ID NO: 1 - SEQ ID NO: 19).

The probes of the invention can be arranged on the solid support in an ordered (known) or non-ordered arrangement. As it is herein used, the term "ordered arrangement" means that the probe is immobilized on the support in a specific location (known position). Therefore, in a particular embodiment, the hybridization platform of the invention comprises two or more probes of the invention immobilized on said solid support in a non-ordered arrangement. Alternatively, in another particular embodiment, the hybridization platform of the invention comprises two or more probes of the invention immobilized on said solid support in an ordered arrangement. Advantageously, the probes of the invention are immobilized on the solid support in an ordered arrangement, forming an array or chip.

DNA biochips or arrays involve the arrangement in isolated areas of a support for a set of specific probes. Since the deposit of the probes is carried out at microscopic points, a very large amount of different probes can be applied in a single biochip, in the order of 10,000-500,000 different probes per biochip, which allows simultaneously detecting different viral pathogens that cause respiratory infections in humans possibly present in a single clinical sample. In addition to detecting and identifying the virus that causes infection, the biochip would also allow quantifying the viral load and even identifying the antiviral drug which it is sensitive or resistant to, for the purpose of facilitating treatment of the patient.

Virtually any method known by persons skilled in the art can be used to immobilize the probes of the invention to the solid support. A commonly used method consists of the noncovalent coating of the solid support with a member of a specific binding pair, for example, streptavidin, and the immobilization of biotinylated oligonucleotide probes. Alternatively, the oligonucleotide probes can be bound directly to the solid support by means of epoxide/amine type coupling reactions.

In a particular embodiment, the solid support is a membrane having a negatively charged surface, such as a nylon membrane. In this case, before binding the probes, the membrane is treated with 1-ethyl-3-(3-dimethylaminopropyl)carbodiimide (EDAC) to activate the carboxyl groups present in the membrane surface. In this case the probes are designed to contain an amino group in the 5' end for the purpose of spatially ordering the probe on the membrane.

In another particular embodiment, the solid support is a glass holder, such as a slide, optionally subjected to treatment with poly-L-lysine for the purpose of arranging on the glass surface amino groups which are going to bind through electrostatic reactions to the phosphate groups of the probes, which are not spatially ordered on the surface.

The concentration of the probes in the solid support may vary within a broad range depending on a number of factors, including the nature and type of solid support, the nature of the probe, etc.; nevertheless, in a particular embodiment, when the solid support is a nylon membrane, the probes can be used at a concentration comprised between approximately 60 and approximately 400 pmoles per lane, preferably, between 0.8 and 1 pmoles/µl. Alternatively, when the solid support is a glass slide, the concentration of the probes can be comprised between approximately 1 and approximately 40 pmol/µl, preferably approximately 20 pmol/µl.

### Method of detection and identification of viruses that cause respiratory infections in humans

In another aspect, the invention relates to a method for identifying viruses that cause respiratory infections in humans, hereinafter method of the invention, selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof, possibly present in an assay sample, comprising:
a) placing nucleic acids from said assay sample in contact with a hybridization platform of the invention comprising a solid support and at least one probe of the invention immobilized on said solid support, where each of said probes is substantially complementary to a specific target sequence of each of said viruses that cause respiratory infection; and
b) detecting the possible hybridization of said target sequences with said probes.

As it is herein used, the term "assay sample" relates to any biological sample suspected of containing one or more viruses that cause respiratory infection in humans, such as influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS) and human metapneumovirus. The genome of said viruses contains target sequences allowing their detection and identification by means of hybridization of said target sequences with the suitable probes, further occasionally enabling their simultaneous typing. The assay sample can come from any biological source, for example, samples of human origin of biological fluids or tissues, such as blood, sputum, gargling, smear (pharyngeal or nasopharyngeal), suctions (nasopharyngeal, bronchial, tracheal or pleural), etc., as well as from blood products (plasma, serum, leukocytes). The assay sample can likewise come from virus cell cultures. The assay sample can be used either directly, as obtained from the source, or after being subjected to a treatment prior to its use for the purpose of modifying its characteristics, for example diluting viscous fluids, inactivating interfering agents, converting double-stranded nucleic acids into single-strand nucleic acids, accumulating, purifying or amplifying target sequences, etc.

Even though the hybridization platform can contain a single probe of the invention, in practice it is preferred that said hybridization platform contains two or more probes of the invention, preferably 2, 3, 4, 5, 6, 7, 8, 9, 10, 11, 12, 13, 14, 15, 16, 17, 18 or 19 of the probes of the invention nucleotide sequences of which are selected from the sequences identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 , SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19. Advantageously, the hybridization platform of the invention comprises said 19 probes of the invention (SEQ ID NO: 1 - SEQ ID NO: 19).

Virtually any probe of the invention, the nucleotide sequence of which is complementary or substantially complementary to a specific target sequence of each of said viruses that cause respiratory infection can be used in the implementation of the method of the invention since the hybridization of the probes with the target sequences is indicative of the presence of the corresponding virus in the analyzed sample. Nevertheless, in a particular embodiment of the method of the invention, the probes of the invention immobilized on said solid support are selected from the group consisting of the polynucleotides identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19. In this case:
the hybridization of a target sequence with a probe selected from the polynucleotides identified as SEQ ID NO: 1, SEQ ID NO: 2 and/or mixtures thereof is indicative of the presence of influenza virus type A in the sample;
the hybridization of a target sequence with a probe selected from the polynucleotides identified as SEQ ID NO: 3, SEQ ID NO: 4 and/or mixtures thereof is indicative of the presence of influenza virus type B in the sample;
the hybridization of a target sequence with the probe identified as SEQ ID NO: 5 is indicative of the presence of influenza virus type C in the sample;
the hybridization of a target sequence with a probe selected from the polynucleotides identified as SEQ ID NO: 6, SEQ ID NO: 7 and/or mixtures thereof is indicative of the presence of human respiratory syncytial virus type A;
the hybridization of a target sequence with the probe identified as SEQ ID NO: 8 is indicative of the presence of human respiratory syncytial virus type B;
the hybridization of a target sequence with the probe identified as SEQ ID NO: 9 is indicative of the presence of human adenovirus;
the hybridization of a target sequence with the probe identified as SEQ ID NO: 10 is indicative of the presence of human parainfluenza virus type 1;
the hybridization of a target sequence with the probe identified as SEQ ID NO: 11 is indicative of the presence of human parainfluenza virus type 2;
the hybridization of a target sequence with the probe identified as SEQ ID NO: 12 is indicative of the presence of human parainfluenza virus type 3;
the hybridization of a target sequence with the probe identified as SEQ ID NO: 13 is indicative of the presence of human parainfluenza virus type 4A and 4B;
the hybridization of a target sequence with the probe identified as SEQ ID NO: 14 is indicative of the presence of enterovirus;
the hybridization of a target sequence with the probe identified as SEQ ID NO: 15 is indicative of the presence of rhinovirus;
the hybridization of a target sequence with the probe identified as SEQ ID NO: 16 is indicative of the presence of human coronavirus type 229E;
the hybridization of a target sequence with the probe identified as SEQ ID NO: 17 is indicative of the presence of human coronavirus type OC43;
the hybridization of a target sequence with the probe identified as SEQ ID NO: 18 is indicative of the presence of coronavirus that causes severe acute respiratory syndrome (SARS); and
the hybridization of a target sequence with the probe identified as SEQ ID NO: 19 is indicative of the presence of human metapneumovirus.

The detection of the hybridization of the target sequences with the probes of the invention can be carried out by means of any suitable method known by persons skilled in the art. The labeling of the genetic material (nucleic acids) to be *hybridized* will be different according to the selected detection method. The label can be incorporated either in the target sequence to be hybridized with the probe or in the actual probe. In a particular embodiment, the detection of the hybridization [step b)] is carried out by means of a radioactive method, whereas in another particular embodiment the detection of the hybridization [step b)] is carried out by means of a non-radioactive method, for example by means of a fluorescent, colorimetric or luminescent (bioluminescent or chemiluminescent) method. By way of illustration, labeling can be carried out with fluorescein or biotin for chemiluminescent detection, or a labeling can be carried out with digoxigenin for chemiluminescent or chemifluorescent detection, etc.

To implement the method of the invention, the viral nucleic acids can be detected and identified without needing to isolate the viral particles.

In a particular embodiment, the method of the invention comprises carrying out a viral nucleic acid amplification reaction before carrying out the hybridization step [step a)]. This alternative is particularly interesting when the concentration of the viruses that cause human respiratory infections to be detected present in respiratory secretions is low (which commonly occurs) since the amount of genetic material (nucleic acid) of the virus containing the target sequence for hybridization with the probe of the invention is also therefore low. To increase its concentration, a nucleic acid amplification reaction can be carried out before carrying out the hybridization step [step a)].

Therefore, in a particular embodiment the invention provides a method for identifying viruses that cause respiratory infections in humans, where said virus are selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof, possibly present in an assay sample, comprising:
(i) placing a nucleic acid from said assay sample in contact with primers flanking specific target sequences of said viruses that cause respiratory infection;
(ii) subjecting the mixture resulting from step (i) to an enzymatic amplification reaction to amplify target sequences of said viruses that cause respiratory infection possibly present in said assay sample;
(iii) placing the amplification products resulting from step (ii) in contact with a hybridization platform of the invention comprising a solid support and at least one probe of the invention, ordered or non-ordered, immobilized on said solid support, where each of said probes is substantially complementary to a specific target sequence of each of said viruses that cause respiratory infection possibly present in the assay sample; and
(iv) detecting the possible hybridization of said target sequences with said probes.

The amplification of a nucleic acid region containing the target sequence can be carried out by means of virtually any target sequence amplification method, such as, for example, polymerase chain reaction (PCR), ligase chain reaction (LCR), band shift amplification [see, in general, G. Walker et al., (1992). Proc. Natl. Acad. Sci. USA 89:392-396; G. Walker et al., (1992). Nucleic Acid Res. 20:1691- 1696], transcription-based amplification [D. Kwoh et al., (1989). Proc. Natl. Acad. Sci. USA 86:1173-1177], self-sustained sequence replication (3SR) [J. Guatelli et al., (1992). Proc. Natl. Acad. Sci. USA 87:1874-1878], Qβ replicase system [P. Lizardi et al., (1988). BioTechnology 6:1197-1202], nucleic acid sequence-based amplification (NASBA) [R. Lewis, (1992). Genetic Engineering News 12(9):1], repair chain reaction (RCR) [R. Lewis, (1992). Genetic Engineering News 12(9):1] and phi29 phage polymerase amplification [L. Blanco et al., (1989) J Biol Chem. 264:8935-8940]. Before the amplification it may be necessary to carry out a reverse transcription or retrotranscription (RT) reaction in order to obtain the cDNA, since most viruses that cause respiratory infection in humans have a genome formed by RNA.

Even though the amplification reaction can be carried out without extracting the nucleic acids contained in the assay sample, said nucleic acids present in the sample are advantageously extracted before carrying out the amplification reaction. Virtually any suitable method for extracting nucleic acids can be used; nevertheless, in a preferred embodiment the nucleic acids contained in the assay sample are extracted by means of using a single step nucleic acid extraction protocol. The genetic material of the sample, both RNA and DNA, is extracted using any of them given that even though virtually all respiratory viruses have an RNA genome, adenoviruses have a DNA genome. Commercial methods such as RNeasy (Qiagen) can be used, although other methods can also be used, for example methods based on extraction with a lysis buffer with a guanidine thiocyanate base [Casas et al., 1995, J Viral Methods 53:25-36].

In a particular embodiment, amplification of the viral nucleic acid regions containing the target sequences (regions intended to be amplified, detected or analyzed) is carried out by means of multiple RT-PCR. To that end, the suitable primers flanking the target sequence to be amplified will be used.

Illustrative examples of primers useful in the RT reaction and in the multiple PCR to amplify the target sequence of the genome of influenza virus types A, B and C, human respiratory syncytial virus types A and B and respiratory adenoviruses are mentioned in Coiras et al., 2003, Journal of Medical Virology 69:132-144.

Illustrative examples of primers useful in the RT reaction and in the multiple PCR to amplify the target sequence of the genome of human parainfluenza virus types 1, 2, 3, 4A and 4B, human coronavirus types 229E and OC43, rhinovirus and enterovirus are mentioned in Coiras et al., 2004, Journal of Medical Virology 72(3):484-495.

Illustrative examples of primers useful for amplifying the target sequence of the genome of the coronavirus that causes severe acute respiratory syndrome (SARS) are mentioned in Drosten et al., 2003, N Engl Med 348(20):1967-1976.

Illustrative examples of primers useful for amplifying the target sequence of the genome of human metapneumoviruses are mentioned in Van Den Hoogen et al., J Infect Dis. 2003 Nov 15; 188(10):1571-7.

A multiple RT-PCR for the simultaneous detection and typing of influenza virus types A, B and C, human respiratory syncytial virus types A and B and a generic detection of human adenoviruses is described in Coiras et al., 2003, J Med Viral 69:132-144.

The features of the assay sample and of the hybridization platform of the invention have already been previously mentioned in relation to the method of the invention (general). Virtually any probe of the invention, the nucleotide sequence of which is complementary or substantially complementary to a specific target sequence of each of said viruses that cause respiratory infection can be used in the implementation of the method of the invention since the hybridization of the probes with the target sequences is indicative of the presence of the corresponding virus in the analyzed sample. Even though the hybridization platform can contain a single probe of the invention, in practice it is preferred that said hybridization platform contains two or more probes of the invention, preferably, 2, 3, 4, 5, 6, 7, 8, 9, 10, 11, 12, 13, 14, 15, 16, 17, 18 or 19 of the probes of the invention the nucleotide sequences of which are selected from the sequences identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 , SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19. The hybridization platform of the invention advantageously comprises said 19 probes of the invention (SEQ ID NO: 1 - SEQ ID NO: 19). Said probes allow the identification of the previously mentioned viruses that cause respiratory infections in humans.

The detection of the hybridization between the target sequences of the viral genome present in the products resulting from the amplification reaction (amplicons) with the probes of the invention can be carried out by means of any of the methods previously mentioned in relation to the detection of the hybridization between the target sequences of the viral genome (unamplified) with the probes of the invention. In a particular embodiment, the detection of the hybridization is carried out by means of a radioactive method, whereas in another particular embodiment it is carried out by means of a non-radioactive method, for example by means of a fluorescent, colorimetric or luminescent (bioluminescent or chemiluminescent) method. By way of illustration, a labeling can be carried out with fluorescein or biotin for chemiluminescent detection, or a labeling can be carried out with digoxigenin for chemiluminescent or chemifluorescent detection, etc. In this case, the labeling can be carried out during the amplification process or subsequently, for example by means of the use of the Klenow polymerase in the presence of aminoallyl-dUTP (Wang et al., 2002, Proc Natl Acad Sci USA. 99(24) :15687-15692) or by using commercial labeling kits such as the CyScribe kits of Amersham Life Sciences.

In a particular embodiment, when the solid support on which the probes of the invention are immobilized is a nylon membrane, it is appropriate to label the genetic material of the sample during the amplification process with biotin bound to a dNTP (either dATP, dUTP, dCGP or dCTP), subsequent treatment with streptavidin-peroxidase and chemiluminescent development with luminol. In another particular embodiment, when the solid support which the probes are bound to is a glass surface, it is advisable to use a fluorescent label, such as Cy3 or Cy5 (Amersham Life Sciences) bound to a dNTP (either dATP, dUTP, dCGP or dCTP), which enables their subsequent detection with a scanner.

Even though the detection systems can be various (radioactive or nonradioactive methods), it is preferable to carry out chemiluminescent detection when the support is a nylon membrane or a slide with a membrane adhered to its surface (for example, Vivid^{™} Gene Array Slides, Pall Life Sciences). To that end, as previously discussed, one of the biotin-labeled nucleotides, for example dCTP (Perkin Elmer Life Sciences) or dUTP (Roche Diagnostics) is added to the PCR reaction used to amplify the starting viral genetic material to obtain biotin-labeled PCR products. After the hybridization reaction between the probes fixed to the support and the denatured strands of the PCR product streptavidin-peroxidase is added at a recommended concentration of 1:4000, which will allow chemiluminescent development by means of the use of luminol in the presence of hydrogen peroxide (for example, ECL Detection System, Amersham Biosciences), according to manufacturer's instructions.

In the case of using a glass support functionalized with, for example, poly-L-lysine, it is advisable to carry out the labeling of the samples using a fluorochrome, for example CyDye fluorochromes of Amersham Biosciences: Cy3 or Cy5. In order to carry out the labeling with this fluorochrome there are kits marketed by Amersham Biosciences for obtaining labeled cDNA or RNA. Nevertheless, the mentioned fluorochromes bound to a nucleotide such as dUTP or dCTP have been used with these probes, carrying out a labeling of the PCR product similar to the one previously mentioned for the case of the labeling of the PCR product with biotin. The reading of the results is carried out using a suitable scanner.

The detection limits were very similar in all the supports used. The quantification of the detection limits has been carried out by means of plasmids containing the fragment resulting from the PCR amplification. The detection limit was 10-100 plasmid copies in all cases.

The method of the invention has a number of advantages compared to other methods used for the detection and identification of viruses, including the following:
- sensitivity exceeding the sensitivity of conventional techniques: isolation in cell cultures, indirect immunofluorescence;
- high specificity;
- scarce dependence on the sample type or on its storage and transport conditions;
- scarce dependence on bacterial contamination of the sample;
- less environmental contamination of amplicons than in genome amplification techniques (therefore it is not necessary to have separate dependencies for the different steps of the technique);
- possibility of simultaneously detecting and identifying (typing) the main viruses that cause respiratory infection in humans;
- easy to routinely implement the technique;
- possibility of adapting to different formats according to laboratory needs (i.e. possibility of adapting to different formats for producing a marketable diagnostic kit); and
- furthermore, as it allows the possibility of detecting coinfections caused by two or more different viruses in the same sample and identifying them, precise epidemiological studies can be carried out, which would aid in determining the contribution of these coinfections to the severity of the infection in the patient, as well as the prognosis of the disease.

Furthermore, the use of oligonucleotide probes for the detection and identification of viruses that cause respiratory infections in humans, such as the probes of the invention, have additional advantages over conventional techniques based on isolation in cell cultures or IF, including the following: (i) in relation to the isolation in cell cultures, the techniques based on hybridization are faster and more sensitive (especially in the case of slow growth viruses or with special growth requirements); and (ii) they are more specific in relation to the techniques based on IF.

### Kits

In another aspect, the invention relates to a kit, hereinafter kit of the invention, for the identification of a virus that causes respiratory infection selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS) and human metapneumovirus, in a biological sample, by means of the method of the invention, comprising at least one probe of the invention, optionally immobilized on a solid support (hybridization platform of the invention). Advantageously, the kit of the invention comprises a hybridization platform of the invention formed by a solid support on which one or more probes of the invention are immobilized. In a particular embodiment, the kit of the invention comprises a hybridization platform comprising a solid support on which probes of the invention have been immobilized selected from the group consisting of the polynucleotides identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 and mixtures thereof. Said probes can be arranged in ordered or non-ordered arrangements.

The kit of the invention includes the reagents necessary for carrying out the method of the invention, as well as instructions to that end. If desired, it may additionally contain primers specific for target sequence amplification (in the event that an amplification reaction is carried out before hybridization) as well as positive and negative controls.

The following examples illustrate the invention and should not be considered to be limiting of the scope thereof.

### EXAMPLE 1

### Detection of reference strains of viral pathogens that cause respiratory infections in samples from cell cultures

The detection of reference strains of viral pathogens that cause respiratory infections in samples from cell cultures inoculated with said viral strains was carried out by means of an assay based on the hybridization of amplified and labeled genetic material fragments with probes immobilized in nylon membrane, according to a protocol adapted from the previously described protocol disclosed by Kaufhold et al. (Kaufhold et al., 1994, FEMS Microbiology Letters 119:19-25).

### 1.1 Materials

### 1.1.1 Samples

The samples used for carrying out this assay were samples from reference strains of respiratory viruses that cause respiratory infections grown in cell cultures. Said reference strains were supplied by prestigious laboratories and are accepted as reference strains by the international scientific community, as listed below:

| **Virus** | **Strain** |
|---|---|
| Influenza virus type A | A/Panama/2007/99 |
| Influenza virus type B | B/Yamanashi/166/98 |
| Influenza virus type C | C/Johannesburg/1 /66 |
| Human respiratory syncytial virus type A (RSVA) | Long |
| Human respiratory syncytial virus type B (RSVB) | RSN-2 |
| Human adenovirus | Serotype 1 (ADV1) |
| Human parainfluenza virus type 1 (PIV1) | C-35 |
| Human parainfluenza virus type 2 (PIV2) | Greer |
| Human parainfluenza virus type 3 (PIV3) | C-243 |
| Human parainfluenza virus type 4B (PIV4B) | M-25 |
| Human coronavirus | 229E |
| Human coronavirus | OC43 |
| Human echovirus 30 (Echo30) | Bastianni |
| Rhinovirus 14 (Rhino14) | 1059 |
| Human metapneumovirus | 00 |

The virus concentrations used were calculated in each case: 100 TCID₅₀ in influenza virus types A and B; a 10⁻³ dilution of a preparation of influenza virus type C with a 2048 titer for inhibition of hemagglutination; a 10⁻³ dilution of RSVA and RSVB, equivalent to detecting an infected cell by indirect immunofluorescence; 10⁻³ dilutions of ADV1, human parainfluenza virus types 1, 2, 3 and 4B, human coronavirus 229E, human coronavirus OC43, and human metapneumovirus, all of them equivalent to 10⁵ plasmid copies; as well as 10⁻⁴ dilutions of Echo30 and Rhinol4, equivalent to 0.1 TCID₅₀.

### 1.1.2 Probes

Probes which are homologous to the template strand of each virus, as well as to the complementary strand, have been used in each case, independently and/or together, for the purpose of checking the best sensitivity result. The concentrations of each probe are those which are previously described. The location of the probes is specified in each case (Figure 1). The probes used in this assay are indicated in Table 1.

**Table 1**

| **Probes used** | |
|---|---|
| **Probe** | **Specificity** |
| SEQ ID NO: 1, SEQ ID NO: 2 and mixtures thereof | Influenza virus type A |
| SEQ ID NO: 3, SEQ ID NO: 4 and mixtures thereof | Influenza virus type B |
| SEQ ID NO: 5 | Influenza virus type C |
| SEQ ID NO: 6, SEQ ID NO: 7 and mixtures thereof | Human respiratory syncytial virus type A |
| SEQ ID NO: 8 | Human respiratory syncytial virus type B |
| SEQ ID NO: 9 | Human adenovirus |
| SEQ ID NO: 10 | Human parainfluenza virus type 1 |
| SEQ ID NO: 11 | Human parainfluenza virus type 2 |
| SEQ ID NO: 12 | Human parainfluenza virus type 3 |
| SEQ ID NO: 13 | Human parainfluenza virus type 4A and 4B |
| SEQ ID NO: 14 | Enterovirus |
| SEQ ID NO: 15 | Rhinovirus |
| SEQ ID NO: 16 | Human coronavirus 229E |
| SEQ ID NO: 17 | Human coronavirus OC43 |
| SEQ ID NO: 19 | Human metapneumovirus |

Said probes have a hexylamino group at the 5' end, as described by Kaufhold et al. (FEMS Microbiol. Lett. 1994 Jun 1; 119(1-2):19-25). The presence of the amino group facilitates the immobilization of the probe and allows spatially ordering the probe on the membrane. For their immobilization on the membrane, the probes were diluted in a recently prepared solution of 500 mM NaHCO₃, pH 8.4, according to a previously described protocol [Vinjé J and Koopmans MPG. J Clin Microbiol 2000, 38:2595-2601]. In this case, all the probes were used at a concentration of 0.8 µM.

### 1.1.3 Membrane

The membrane used in this assay was a membrane with a negative surface charge (Biodyne C, Pall Life Sciences) having a size of about 14.5 cm x 14.5 cm. Said membrane was prepared according to a previously described protocol [Vinjé and Koopmans mentioned above]. Before binding the probes to the membrane, the latter was briefly treated with a recently prepared solution of 16% 1-ethyl-3-(3-dimethylaminopropyl) carbodiimide (EDAC) in water for at least 15 minutes, and was then rinsed with water. The membrane thus prepared was used immediately or was stored at 4°C in filter paper until its use for a maximum period of 1 week.

### 1.2 Methods

### 1.2.1 Extraction of genetic material

The genetic material, both RNA and DNA, was extracted from the samples (section 1.1.1) by means of conventional methods. In this case an extraction method based on the use of guanidinium thiocyanate was used, previously described by Casas et al. (Casas et al., 1995, J Virol Methods 53:25-36).

### 1.2.2 Enzymatic amplification

The extracted genetic material was subjected to an enzymatic amplification reaction for the purpose of amplifying fragments that contained the sequences that are homologous to the probes used (Table 1) for the detection of the different viral pathogens (influenza virus types A, B and C, RSVA, RSVB, ADV1, PIV1, PIV2, PIV3, PIV4, coronavirus, echovirus, rhinovirus and metapneumovirus) immobilized on a nylon membrane.

The enzymatic amplification reactions of the different fragments were carried out using the primers described by Coiras et al. (Coiras et al., Journal of Medical Virology 69:132-144 (2003); and Coiras et al., Journal of Medical Virology 72(3):484-495 (2004)). The amplification was carried out in the presence of 200 µM dATP, dGTP and dCTP, 130 µM dTTP and 70 µM biotin-16-dUTP, by reaction for the purpose of labeling the genetic material (amplification products or amplicons) to be hybridized.

### 1.2.3 Immobilization of the probes to the nylon membrane

The immobilization of the probes used in this assay (Table 1) to the nylon membrane was carried out substantially according to a previously described protocol [Vinjé J, Koopmans MPGJ Clin Microbiol 2000, 38:2595-2601].

For their immobilization on the membrane, the probes were diluted in a recently prepared solution of 500 mM NaHC0₃, pH 8.4, according to the previously described protocol. Likewise, before binding the probes to the nylon membrane, the latter was treated with a recently prepared solution of 16% 1-ethyl-3-(3-dimethylaminopropyl) carbodiimide (EDAC) in water for at least 15 minutes and was then rinsed with water. The membrane thus prepared was used immediately or was stored at 4^{°C} in filter paper until its use for a maximum period of 1 week.

The probes subjected to the previously indicated treatment were immobilized on the nylon membrane previously prepared by conventional methods [Vinjé and Koopmans mentioned above] based on the fact that the amino group present in the probes covalently binds to the activated carboxyl groups coming from the treatment of the membrane with EDAC. To that end, the nylon membrane was briefly introduced in a 45-channel miniblotter (Biometra). The channels which were not going to contain probe were filled with a recently prepared solution of 500 mM NaHCO₃, pH 8.4, whereas the channels which were going to contain the probes were filled with dilutions of the probes prepared at the time of use as previously indicated. The probes were incubated in contact with the membrane at room temperature (18-22°C) for 10 minutes. The liquid was then suctioned from the channels with a vacuum pump. The ester groups which remained activated in the membrane were hydrolyzed by means of incubation at room temperature with a 100 mM NaOH solution for a time period of not more than 10 minutes. The membrane with the probes was finally rinsed several times with ultrapure distilled water. The concentration of the probes in the nylon membrane was approximately 0.8-5 pmoles/µl (or 120-750 pmoles per lane) in a final volume of 150 µl, which completely filled the lane of the miniblotter (Biometra). The probes were arranged on the membrane always according to the same order in the lanes. The results were analyzed by means of a template which allowed quickly identifying the viruses that had been detected in the sample (s) .

### 1.2.4 Hybridization of the amplified and labeled genetic material with the probes immobilized in the nylon membrane

Before hybridizing the labeled genetic material (amplification product), the membrane with the probes was subjected to a pre-hybridization treatment. To that end, the membrane with the probes was washed with a 2X SSPE solution (360 mM NaCl, 20 mM NaH₂PO₄, 2 mM EDTA, pH 7.2) in the presence of 0.1% sodium dodecyl sulfate (SDS) for 10 minutes at 56^{°C}.

For the hybridization of the genetic material with the probes immobilized, the membrane with the probes was placed in a miniblotter rotated 90° with respect to the previous position. Ten microliters of the labeled genetic material were diluted in 150 µl of hybridization buffer (2x SSPE, 0.5% SDS). Given that the product to be hybridized is double-stranded DNA, it was subjected for 10 minutes to a heating at 99°C and was then quickly cooled for 1 minute at 4°C. Hybridization was then carried out after maintaining it at room temperature for 1 minute. The hybridization process was carried out for approximately 2 hours, at approximately 48°C, under gentle stirring.

The membrane was then washed through the filtration manifolds (Biometra) with 150 ml of a 2x SSPE solution with 0.5% SDS (sodium dodecyl sulfate). The membrane was removed from the miniblotter and was washed two more times with 50 ml of the previous solution, under stirring, for 10 minutes each time, at 40°C.

### 1.2.5 Detection and identification of the viral pathogens

The nylon membrane with the probes and the labeled amplification products, which had eventually hybridized with the probes, was incubated with streptavidin-peroxidase (1:4000) for a time period of 45 minutes, at 37°C. Three washings of 10 minutes each were then carried out, using in the first two a solution of 2x SSPE 0.5% SDS and 2x SSPE 0.1% SDS, respectively, at 37°C and under stirring, and the third one with a solution of 2x SSPE alone, at room temperature, under stirring. Chemiluminescent development was finally carried out with luminol in the presence of hydrogen peroxide using a commercial kit (ECL Detection System, Amersham Biosciences), according to the manufacturer's instructions. The results were viewed by means of X-ray film printing (Kodak) for 5 minutes, 30 minutes and 4 hours.

### 1.3 Results

The obtained results are shown in Figure 1 and clearly show the specificity and sensitivity of the assayed technique which allows simultaneously identifying the prototype strains of different viral pathogens that cause respiratory infections. Each biotin-labeled amplification product specifically interacts only with its complementary probe, and in no case were unspecific hybridizations observed despite the fact that the amount of mold used for the amplification reaction was high. By means of applying a template on the printed film it is possible to quickly and clearly identify the virus/viruses which has/have been detected by the technique.

### EXAMPLE 2

### Detection of respiratory viruses isolated from clinical samples of the respiratory tract inoculated in cell cultures

The detection of different respiratory viruses that cause respiratory infections in samples from cell cultures was carried out by means of an assay based on the hybridization to the probes immobilized in a nylon membrane of the amplified and biotin-labeled genetic material fragments.

### 2.1 Materials

### 2.1.1 Samples

The samples used for carrying out this assay were samples of the respiratory viruses that cause respiratory infections, isolated from clinical samples of the respiratory tract inoculated in cell cultures. Said viruses were the following: influenza virus type A, influenza virus type B, human respiratory syncytial virus type A (RSVA), human respiratory syncytial virus type B (RSVB), human adenovirus (ADV) (several serotypes) and human parainfluenza virus type 3 (PIV3).

For the purpose of checking the sensitivity of the technique serial ten-fold dilutions of viruses isolated in cell cultures from respiratory samples of patients were used. The dilutions used for each virus were the following: the initial dilutions, marked as -1 (Figure 2), were 100 TCID₅₀ in the influenza virus types A (lanes 1-4) and B (lanes 5-8), a 10⁻³ dilution of RSVA (lanes 9-12) and RSVB (lanes 13-16), ADV (lanes 17-20), equivalent to detecting an infected cell by indirect immunofluorescence, and a 10⁻³ dilution of human parainfluenza virus type 3 (lanes 21-24), equivalent to detecting 10⁵ copies of a plasmid containing the amplified fragment by RT-PCR.

### 2.1.2 Probes

Probes which are homologous to the template strand of each virus, as well as to the complementary strand, have been used in each case, independently and/or together, for the purpose of checking the best sensitivity result. The probes used in this assay are indicated in Table 2.

**Table 2**

| **Probes used** | |
|---|---|
| **Probe** | **Specificity** |
| SEQ ID NO: 1 and SEQ ID NO: 2 | Influenza virus type A |
| SEQ ID NO: 3 and SEQ ID NO: 4 | Influenza virus type B |
| SEQ ID NO: 6 and SEQ ID NO: 7 | Human respiratory syncytial virus type A |
| SEQ ID NO: 8 | Human respiratory syncytial virus type B |
| SEQ ID NO: 9 | Human adenovirus |
| SEQ ID NO: 12 | Human parainfluenza virus type 3 |

The probes designed for the detection of influenza virus types A and B and RSVA were used together or separately, at a final concentration of 0.8 µM. The remaining probes were used at concentrations of 5, 1 and 0.8 µM for the purpose of observing the different sensitivity according to the variation of the concentration.

Said probes have a hexylamino group at the 5' end, as described by Kaufhold et al. (FEMS Microbiol Lett. 1994 Jun 1;119(1-2): 19-25). The presence of the amino group facilitates the immobilization of the probe and allows spatially ordering the probe on the membrane. For their immobilization on the membrane, the probes were diluted in a recently prepared solution of 500 mM NaHCO₃, pH 8.4, according to a previously described protocol [Vinjé J and Koopmans MPG. J Clin Microbiol 2000, 38:2595-2601].

### 2.1.3 Membrane

The membrane used in this assay was a membrane with a negative surface charge (Biodyne C, Pall Life Sciences), having a size of about 14.5 cm x 14.5 cm. Said membrane was prepared according to a previously described protocol [Vinjé and Koopmans mentioned above]. Before binding the probes to the membrane, the latter was briefly treated with a recently prepared solution of 16% EDAC in water for at least 15 minutes and was then rinsed with water. The membrane thus prepared was used immediately or was stored at 4°C in filter paper until its use for a maximum period of 1 week.

### 2.2 Methods

### 2.2.1 Extraction of genetic material

The genetic material, both RNA and DNA, was extracted from the samples (section 2.1.1.) by means of conventional methods. In this case an extraction method based on the use of guanidinium thiocyanate was used, previously described by Casas et al. (J Virol Methods, 1995, 53:25-36).

### 2.2.2 Enzymatic amplification

The extracted genetic material was subjected to an enzymatic amplification reaction for the purpose of amplifying fragments that contained the sequences that are homologous to the probes used (Table 2) for the detection of the different viral pathogens (influenza virus types A and B, RSVA, RSVB, ADV and human parainfluenza virus type 3), immobilized on a nylon membrane.

The enzymatic amplification reactions of the different specific fragments of the viral pathogens influenza virus types A and B, RSVA, RSVB, ADV and human parainfluenza virus type 3 were carried out using the primers described by Coiras et al. (Coiras et al., Journal of Medical Virology 69:132-144 (2003); and Coiras et al., Journal of Medical Virology 72(3):484-495 (2004)). In all cases, the amplification was carried out in the presence of 200 µM dATP, dGTP, and dCTP, 130 µM dTTP and 70 µM biotin-16-dUTP, by reaction for the purpose of labeling the genetic material (amplification products or amplicons) to be hybridized.

### 2.2.3 Immobilization of the probes to the nylon membrane

The immobilization of the probes used in this assay (Table 2) to the nylon membrane was carried out in a manner that is substantially identical to that mentioned in Example 1.2.3.

### 2.2.4 Hybridization of the amplified genetic material with the probes immobilized in the nylon membrane

The hybridization of the amplified genetic material with the probes immobilized in the nylon membrane was carried out in a manner that is substantially identical to that mentioned in Example 1.2.4.

### 2.2.5 Detection and identification of the viral pathogens

The detection and identification of the viral pathogens was carried out in a manner that is substantially identical to that mentioned in Example 1.2.5.

### 2.3 Results

The obtained results are shown in Figure 2 and clearly show the specificity and sensitivity of the assayed technique which allows identifying different viral pathogens that cause respiratory infections, isolated from clinical samples of the respiratory tract inoculated in cell cultures. The variation in the sensitivity of the detection according to the dilution of the virus and of the different concentration of the probe used, as well as if the probes for the detection of influenza virus types A and B and of the RSV type A are used together or separately, can be observed. In all cases, specificity is very high.

### EXAMPLE 3

### Detection of influenza virus type A present in clinical samples

The direct detection of influenza virus type A present in the assayed clinical sample was carried out by means of an assay based on the hybridization of amplified and labeled genetic material fragments with probes immobilized in nylon membrane.

### 3.1 Materials

### 3.1.1 Samples

The samples used for carrying out this assay were pharyngeal smears taken from patients who presented symptoms indicating the flu, for the purpose of trying to identify the virus which causes said clinical signs.

### 3.1.2 Probes

The probes used in this assay (SEQ ID NO: 1 and SEQ ID NO: 2) were designed with a hexylamino group at the 5' end, as described by Kaufhold et al. (FEMS Microbiol Lett. 1994 Jun 1;119(1 -2):19-25). The presence of the amino group facilitates the immobilization of the probe and allows spatially ordering the probe on the membrane. For their immobilization on the membrane, the probes were diluted in a recently prepared solution of 500 mM NaHC0₃, pH 8.4, according to a previously described protocol [Vinjé J and Koopmans MPG. J Clin Microbiol 2000, 38:2595-2601]. In this case, all the probes were used at a concentration of 0.8 µM.

### 3.1.3 Membrane

The membrane used in this assay was a membrane with a negative surface charge (Biodyne C, Pall Life Sciences) having a size of about 14.5 cm x 14.5 cm. Said membrane was prepared according to a previously described protocol [Vinjé and Koopmans mentioned above]. Before binding the probes to the membrane, the latter was briefly treated with a recently prepared solution of 16% EDAC in water, for at least 15 minutes, and was then rinsed with water. The membrane thus prepared was used immediately or was stored at 4°C in filter paper until its use for a maximum period of 1 week.

### 3.2 Methods

### 3.2.1 Extraction of genetic material

The genetic material, both RNA and DNA, was extracted from the samples (section 3.1.1) by means of conventional methods In this case an extraction method based on the use of guanidinium thiocyanate was used, previously described by Casas et al. (Casas et al., 1995, J Viral Methods 53:25-36).

### 3.2.2 Enzymatic amplification

The extracted genetic material was subjected to an enzymatic amplification reaction for the purpose of amplifying fragments that contained the sequences that are homologous to the probes used (SEQ ID NO: 1 and SEQ ID NO: 2) for the detection of the viral pathogen (influenza virus type A) fixed to a nylon membrane. The enzymatic amplification reaction (RT-PCR) of the different fragments was carried out using the primers and the conditions described by Coiras et al. (Coiras et al., Journal of Medical Virology 69:132-144 (2003)). In all cases, the amplification was carried out in the presence of 200 µM dATP, dGTP, and dCTP, 130 µM dTTP and 70 µM biotin-16-dUTP by reaction for the purpose of labeling the genetic material (amplification products or amplicons) to be hybridized.

### 3.2.3 Immobilization of the probes to the nylon membrane

The immobilization of the probes used in this assay (SEQ ID NO: 1 and SEQ ID NO: 2) to the nylon membrane was carried out in a manner that is substantially identical to that mentioned in Example 1.2.3.

### 3.2.4 Hybridization of the amplified genetic material with the probes immobilized in the nylon membrane

The hybridization of the amplified genetic material with the probes immobilized in the nylon membrane was carried out in a manner that is substantially identical to that mentioned in Example 1.2.4.

### 3.2.5 Detection and identification of the viral pathogen

The detection and identification of the viral pathogen was carried out in a manner that is substantially identical to that mentioned in Example 1.2.5.

### 3.2.6 Nested-PCR

For the purpose of checking the previously used technique based on the hybridization of amplified and labeled products with probes immobilized in a nylon membrane, nested-PCR was carried out according to a previously described methodology (Coiras et al., Journal of Medical Virology, 2003, 69:132-144). The primers used for the amplification of the fragments that contained sequences homologous to the probes, and the ones used to carry out nested-PCR, as well as the conditions for carrying out the nested-PCR, are described in said publication (Coiras et al., 2003, mentioned above).

### 3.3 Results

The results of the hybridization of the genetic material from the virus of the clinical sample, with the probes immobilized in the membrane (Lanes 1-12), can be observed in Figure 3A. Influenza virus A was not detected in samples 2, 7 and 8. These results clearly show the specificity and sensitivity of the assayed technique which allows identifying influenza virus type A in clinical samples.

For the purpose of analyzing this sensitivity and specificity using a referenced technique, nested-PCR was carried out at the same time as the hybridization process, the results of which are shown in Figure 3B, where lanes 1-12 correspond to lanes 1-12 observed in Figure 3A. Lane M of Figure 3B corresponds with molecular weight marker XIV, supplied by Roche Diagnostics (marker of 100 bp). A band of 721 base pairs (bp), corresponding to the amplified fragment of the gene of the nucleoprotein of influenza virus type A, and a band of 837 bp, corresponding to the internal control used as a control of the process for extracting the genetic material from the sample, and of the PCR reaction(the plasmid of the internal control and the primers for its amplification are described in Coiras et al., (2003) mentioned above, where the primers and the conditions for carrying out nested-PCR are also described), can be observed in all the lanes (except 2, 7 and 8).

### EXAMPLE 4

### Detection of the coronavirus that causes severe acute respiratory syndrome (SARS)

The detection of the coronavirus that causes severe acute respiratory syndrome (SARS) was carried out by means of an assay based on the hybridization of amplified and labeled genetic material fragments with probes immobilized in nylon membrane.

### 4.1 Materials

### 4.1.1 Samples

The samples used for carrying out this assay were genetic material from an inactivated virus and from a plasmid including a fragment of the coronavirus polymerase that causes severe acute respiratory syndrome (SARS, kindly supplied by Dr. Christian Drosten (Bernhard Nocht Institute for Tropical Medicine, National Reference Center for Tropical Infectious Diseases, Hamburg, Germany). Serial dilutions of the inactivated virus of the 10⁻³ dilution, equivalent to serial dilutions carried out from a 10⁵ dilution of plasmid including the fragment of the gene of the polymerase amplified from the CoV-SARS genome, have been used.

### 4.1.2 Probes

The probe used in this assay (SEQ ID NO: 18) was designed with a hexylamino group at the 5' end, as described by Kaufhold et al. (FEMS Microbiol Lett. 1994 Jun 1 ;119(1-2):19-25). The presence of the amino group facilitates the immobilization of the probe and allows spatially ordering the probe on the membrane. Two concentrations of said probe (0.8 µM and 5 µM) were assayed, as included in Figure 4. For their immobilization on the membrane, the probes were diluted in a recently prepared solution of 500 mM NaHCO₃, pH 8.4, according to a previously described protocol [Vinjé J and Koopmans MPG. J Clin Microbiol 2000, 38:2595-2601].

### 4.1.3 Membrane

The membrane used in this assay was a membrane with a negative surface charge (Biodyne C, Pall Life Sciences) having a size of about 14.5 cm x 14.5 cm. Said membrane was prepared according to a previously described protocol [Vinjé and Koopmans mentioned above]. Before binding the probes to the membrane, the latter was briefly treated with a recently prepared solution of 16% EDAC in water for at least 15 minutes and was then rinsed with water. The membrane thus prepared was used immediately or was stored at 4°C in filter paper until its use for a maximum period of 1 week.

### 4.2 Methods

### 4.2.1 Extraction of genetic material

The genetic material, both RNA and DNA, was extracted from the samples (section 4.1.1) by means of conventional methods. In this case an extraction method based on the use of guanidinium thiocyanate was used, previously described by Casas et al. (1995, J Virol Methods 53:25-36).

### 4.2.2 Enzymatic amplification

The extracted genetic material was subjected to an enzymatic amplification reaction for the purpose of amplifying fragments that contained the sequences that are homologous to the probes used (SEQ ID NO: 18) for the detection of the viral pathogen (coronavirus that causes severe acute respiratory syndrome (SARS)) fixed to a nylon membrane. The enzymatic amplification reaction of the different fragments was carried out using the primers and the conditions described by Drosten C et al. (Drosten C et al., 2003, N Engl J Med. May 15;348(20): 1967-76). The amplification was carried out in the presence of 200 µM dATP, dGTP, and dCTP, 130 µM dTTP and 70 uM biotin-16-dUTP, by reaction for the purpose of labeling the genetic material (amplification products or amplicons) to be hybridized.

### 4.2.3 Immobilization of the probes to the nylon membrane

The immobilization of the probe used in this assay (SEQ ID NO: 18) to the nylon membrane was carried out in a manner that is substantially identical to that mentioned in Example 1.2.

### 3.4.2.4 Hybridization of the amplified genetic material with the probes immobilized in the nylon membrane

The hybridization of the amplified genetic material with the probe immobilized in the nylon membrane was carried out in a manner that is substantially identical to that mentioned in Example 1.2.4.

### 4.2.5 Detection and identification of the viral pathogen

The detection and identification of the viral pathogen was carried out in a manner that is substantially identical to that mentioned in Example 1.2.5.

### 4.3 Results

The obtained results are shown in Figure 4 and clearly show the validity of the probe used for the detection of the genetic material from the coronavirus that causes severe acute respiratory syndrome (SARS) and the sensitivity of the assay.

### EXAMPLE 5

### Detection of human adenovirus present in clinical samples

The generic detection of human adenoviruses present in clinical samples was carried out by means of an assay based on the hybridization of amplified and labeled genetic material fragments with probes immobilized, in a non-ordered arrangement, in a glass slide.

### 5.1 Materials

### 5.1.1 Samples

The samples used for carrying out this assay were respiratory secretions from patients with respiratory symptoms. The genetic material was extracted directly from the clinical samples and the possible virus present therein was analyzed by means of two multiple RT-PCRs followed by nested-PCRs previously described by Coiras et al. (Coiras et al., Journal of Medical Virology, 2003, 69:132-144; and Coiras et al., Journal of Medical Virology, 2004, 72(3):484-495). Once the presence of human adenoviruses in said clinical samples is known, the same genetic material was used to be hybridized with the probe SEQ ID NO: 9, designed to be hybridized to any adenovirus of human origin, independently of its serotype.

### 5.1.2 Probes

The probe used in this assay (SEQ ID NO: 9) was immobilized directly on a glass surface (slide), in a non-ordered spatial arrangement, at a concentration of 20 µM. The probes were prepared at the recommended concentration in a volume of about 40 µl diluted in 3x SSC, arranged in the wells of a 96-well plate, preferably having a U or V-shaped bottom to facilitate access of the print needles.

Unlike the method of hybridization in a nylon membrane, these probes have no modifications at the 5' end.

### 5.1.3 Glass support

Common slides subjected to treatment with poly-L-lysine for the purpose of arranging on the glass surface amino groups which are going to covalently bind to the phosphate groups of the probes, have been used. The probes are not spatially ordered on the surface of the slide functionalized with poly-L-lysine, but rather they adhere to it along their entire length. This treatment was described by (Schena et al., 1995, Science 270(5235):467-470.).

### 5.2 Methods

### 5.2.1 Extraction of genetic material

The genetic material, both RNA and DNA, was extracted from the samples (section 5.1.1) by means of conventional methods. In this case an extraction method based on the use of guanidinium thiocyanate was used, previously described by Casas et al. (J Viral Methods, 1995, 53:25-36).

### 5.2.2 Enzymatic amplification

The extracted genetic material was subjected to an enzymatic amplification reaction for the purpose of amplifying fragments of the sequence of the gene of the hexon protein, homologous to the probe used (SEQ ID NO: 9) for the detection of adenovirus fixed to a nylon membrane. The enzymatic amplification reaction was carried out using the primers and the conditions described in Coiras et al. (Journal of Medical Virology, 2003, 69:132-144). The amplification was carried out in the presence of 200 µM dATP, dGTP, and dTTP, 180 µM dCTP and 20 µM Cy3-dCTP by reaction for the purpose of labeling the genetic material (amplification products or amplicons) to be hybridized.

### 5.2.3 Immobilization of the probes to the glass support

The immobilization of the probe used in this assay (SEQ ID NO: 9) to the glass surface was carried out by arranging said probe in triplicate on the slide by means of a Pin and Ring system with a GMS 417 Arrayer (Affymetrix Inc.). The amino groups which are free on the surface of the functionalized and printed slide are blocked with succinic anhydride dissolved in a polar solvent (n-methylpyrrolidinone), as described by Eisen and Brown, 1999, Methods Enzymol 303:179-205. The slide was then immersed in water at 90°C for 2 minutes and then momentarily in absolute ethanol for the purpose of facilitating the drying thereof, which is completed by means of centrifuging at room temperature. Once they are blocked, the slides are stored in a dryer, protected from light, at room temperature, at least 24 hours before carrying out hybridization with the samples.

### 5.2.4 Hybridization of the amplified genetic material with the probes immobilized in the glass support

The hybridization of the amplified and labeled genetic material with the probe immobilized in the glass support was carried out by means of the following protocol. Briefly, 10 µl of the labeled genetic material were diluted in 15-20 µl of hybridization buffer (2x SSC, 0.2% SDS), heated at 95°C for 10 minutes and then quickly cooled for 1 minute at 4°C. The sample was deposited on the region of the slide where the probes were printed and was covered with a cover slip. It was then introduced in a hybridization chamber where it was maintained under moisture and salt saturation conditions, and hybridization was carried out for 2 hours at 42°C under gentle stirring.

### 5.2.5 Detection and identification of the viral pathogen

The detection and identification of the viral pathogen was carried out after carrying out washings of the slides with 1x SSC -0.2% SDS, 0.1x SSC -0.2% SDS, and finally with 0.1x SSC, for 10 minutes each, under stirring, at room temperature and protected from light. After drying them in a slide centrifuge, the results were read immediately.

### 5.3 Results

The results were read using a GMS 418 Scanner (Affymetrix Inc.). The obtained results are shown in Figure 5 and clearly show the validity of the probe used for the detection of the genetic material from clinical samples infected with any adenovirus of human origin. Figure 5 shows the fluorescence emitted by the Cy3 molecules present in the amplification product obtained by means of the primer oligonucleotides specific for human adenovirus. Dilutions of human adenoviruses that cause respiratory infection and belonging to serotypes 1 (image 1), 2 (image 2), 4 (image 3), 5 (image 4) and 7 (image 5), these dilutions being equivalent to 10³ copies of a plasmid containing the fragment of the gene of the hexon protein amplified under the described conditions, have been used for the amplification and labeling by means of PCR (conditions and oligonucleotides in Coiras et al., Journal of Medical Virology 69:132-144 (2003)).

## Claims

1. A probe having a nucleotide sequence selected from the group consisting of the sequences identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ-ID NO: 8, SEQ ID NO: 9, SEQ ID NO: 10 , SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18 and SEQ ID NO: 19.

2. A hybridization platform comprising a solid support and at least one probe according to claim 1, immobilized on said solid support.

3. A hybridization platform according to claim 2, comprising a set of two or more of said probes immobilized on said solid support in an ordered arrangement.

4. A hybridization platform according to claim 2, comprising a set of two or more of said probes immobilized on said solid support in a non-ordered arrangement.

5. A method for identifying viruses that cause respiratory infections in humans selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and mixtures thereof, eventually present in an assay sample, comprising:
a) placing nucleic acids from said assay sample in contact with a hybridization platform comprising a solid support and at least one probe immobilized on said support, where each probe is substantially complementary to a specific target sequence of each of said viruses that cause respiratory infection eventually present in said assay sample; and
b) detecting the possible hybridization of said target sequences with said probes.

6. A method according to claim 5, wherein said probes immobilized on said solid support are selected from the group consisting of the polynucleotides identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 and mixtures thereof, and wherein
the hybridization of a target sequence with a probe selected from the polynucleotides identified as SEQ ID NO: 1, SEQ ID NO: 2 and/or mixtures thereof is indicative of the presence of influenza virus type A in the sample;
the hybridization of a target sequence with a probe selected from the polynucleotides identified as SEQ ID NO: 3, SEQ ID NO: 4 and/or mixtures thereof is indicative of the presence of influenza virus type B in the sample;
the hybridization of a target sequence with the probe identified as SEQ ID NO: 5 is indicative of the presence of influenza virus type C in the sample;
the hybridization of a target sequence with a probe selected from the polynucleotides identified as SEQ ID NO: 6, SEQ ID NO: 7 and/or mixtures thereof is indicative of the presence of human respiratory syncytial virus type A;
the hybridization of a target sequence with the probe identified as SEQ ID NO: 8 is indicative of the presence of human respiratory syncytial virus type B;
the hybridization of a target sequence with the probe identified as SEQ ID NO: 9 is indicative of the presence of human adenovirus;
the hybridization of a target sequence with the probe identified as SEQ ID NO: 10 is indicative of the presence of human parainfluenza virus type 1;
the hybridization of a target sequence with the probe identified as SEQ ID NO:. 11 is indicative of the presence of human parainfluenza virus type 2;
the hybridization of a target sequence with the probe identified as SEQ ID NO: 12 is indicative of the presence of human parainfluenza virus type 3;
the hybridization of a target sequence with the probe identified as SEQ ID NO: 13 is indicative of the presence of human parainfluenza virus type 4A and 4B;
the hybridization of a target sequence with the probe identified as SEQ ID NO: 14 is indicative of the presence of enterovirus;
the hybridization of a target sequence with the probe identified as SEQ ID NO: 15 is indicative of the presence of rhinovirus;
the hybridization of a target sequence with the probe identified as SEQ ID NO: 16 is indicative of the presence of human coronavirus type 229E;
the hybridization of a target sequence with the probe identified as SEQ ID NO: 17 is indicative of the presence of human coronavirus type OC43;
the hybridization of a target sequence with the probe identified as SEQ ID NO: 18 is indicative of the presence of coronavirus that causes severe acute respiratory syndrome (SARS); and
the hybridization of a target sequence with the probe identified as SEQ ID NO: 19 is indicative of the presence of human metapneumovirus.

7. A method according to claim 5, wherein said assay sample is a sample suspected of containing one or more viruses that cause respiratory infection in humans selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and mixtures thereof.

8. A method according to claim 7, wherein said assay sample is a biological sample of human origin or a sample from a virus cell culture.

9. A method according to claim 8, wherein said biological sample of human origin is a sample of a biological fluid or a sample of a biological tissue.

10. A method according to claim 9, wherein said biological sample of human origin is selected from blood, sputum, gargling, pharyngeal smear, nasopharyngeal smear and nasopharyngeal, bronchial, tracheal or pleural suction.

11. A method according to claim 5, wherein said solid support is a nylon membrane or a glass surface.

12. A method according to claim 5, wherein said probes are arranged on the solid support in an ordered arrangement.

13. A method according to claim 5, wherein said probes are arranged on the solid support in a non-ordered arrangement.

14. A method according to claim 5, wherein said probes are arranged on the solid support forming an array.

15. A method according to claim 5, wherein said solid support is a biochip in which the immobilized probes are arranged in an ordered arrangement forming an array.

16. A method according to claim 5, wherein the detection of the hybridization [step b)] is carried out by means of a radioactive method.

17. A method according to claim 5, wherein the detection of the hybridization [step. b.)] is carried out by means of a non-radioactive method.

18. A method according to claim 17, wherein said non-radioactive method is a fluorescent, colorimetric, luminescent, bioluminescent or chemiluminescent method.

19. A method according to claim 5, wherein, before carrying out step a), an amplification reaction of the nucleic acids of said viruses that cause respiratory infection in humans eventually present in the assay sample is carried out, using primers flanking specific target sequences of each of said viruses that cause respiratory infection.

20. A method according to claim 19, wherein said amplification reaction comprises a multiplex polymerase chain reaction (PCR) or a multiplex reverse transcription (RT) - PCR reaction.

21. A kit for the identification of a virus that causes respiratory infection selected from influenza virus type A, influenza virus type B, influenza virus type C, human respiratory syncytial virus type A, human respiratory syncytial virus type B, human adenovirus, human parainfluenza virus type 1, human parainfluenza virus type 2, human parainfluenza virus type 3, human parainfluenza virus types 4A and 4B, enterovirus, rhinovirus, human coronavirus type 229E, human coronavirus type OC43, coronavirus that causes severe acute respiratory syndrome (SARS), human metapneumovirus and combinations thereof, in an assay sample by means of a method according to claim 5, comprising at least one probe according to claim 1, or at least one hybridization platform according to any of claims 2 to 4.

22. A kit according to claim 21, comprising a hybridization platform comprising a solid support on which a probe has been immobilized, selected from the group consisting of the polynucleotides identified as SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 and mixtures thereof.
